# EUROPÄISCHE PATENTSCHRIFT

(11) **EP 3 708 133 B1**
(45) Veröffentlichungstag und Bekanntmachung des Hinweises auf die Patenterteilung: **01.05.2024**
(21) Anmeldenummer: 19162395.8
(22) Anmeldetag: 12.03.2019
(51) Int. Cl.: A61F 9/02, G02C 7/02

(54) **SCHUTZBRILLE FÜR EINEN MENSCHLICHEN BENUTZER**
PROTECTIVE EYEWEAR FOR A HUMAN USER
LUNETTES PROTECTRICES POUR UN UTILISATEUR HUMAIN

(43) Veröffentlichungstag der Anmeldung: 16.09.2020
(73) Patentinhaber: Moldex/Metric AG & Co. KG, 72141 Walddorfhäslach (DE)
(72) Erfinder: SKOV, Torben, 72124 Pliezhausen (DE); SKOV, Roman, 70197 Stuttgart (DE)
(74) Vertreter: Clarenbach, Carl-Philipp

(56) Entgegenhaltungen:
- WO-A1-2008/072119
- WO-A1-2018/117943
- US-A- 5 825 455

## Beschreibung

Die Erfindung betrifft eine Schutzbrille für einen menschlichen Benutzer, mit einer Brillenscheibe, die einen der Stirn des Benutzers zuordenbaren oberen Rand und einen der Wange des Benutzers zuordenbaren unteren Rand sowie für jedes Auge des Benutzers jeweils einen Brillenscheibenabschnitt, der sich jeweils entlang zumindest einer Krümmungslinie erstreckt, aufweist.

Weiterhin betrifft die Erfindung ein Schutzsystem für einen menschlichen Nutzer, mit einer Atemschutzmaske und mit einer Schutzbrille.

Schutzbrillen der eingangs genannten Art sind aus dem Stand der Technik bekannt. Sie dienen dazu, die Augen eines Benutzers vor Staub, Späne, Flüssigkeit, Temperatur oder anderer Produkte oder Elemente zu schützen, die insbesondere bei der Bearbeitung eines Werkstücks entstehen und in Richtung des Benutzers geschleudert werden oder wirken können. Üblicherweise weist eine Schutzbrille eine Brillenscheibe aus einem durchsichtigen Material auf, das einen Brechungsindex einer herkömmlichen Glasscheibe aufweist und insofern die Optik des Benutzers nicht oder kaum beeinträchtigt. Die Brillenscheibe weist dabei regelmäßig zwei Brillenscheibenabschnitte auf, wobei jeweils ein Brillenscheibenabschnitt einem Auge des Benutzers zuordenbar ist, sodass jedem Auge des Benutzers ein eigener Brillenscheibenabschnitt zugehörig ist. Die Brillenscheibenabschnitte erstrecken sich häufig entlang zumindest einer Krümmungslinie, um eine ergonomische vorteilhafte Anpassung des Verlaufs der Brillenscheibe an das Gesicht des Benutzers zu gewährleisten. Unter einer Krümmungslinie ist dabei eine in zumindest einer Dimension gekrümmte Linie zu verstehen, entlang welcher sich die Brillenscheibe erstreckt. Die Krümmungslinie gibt somit die Krümmung der Brillenscheibe in Längserstreckung der Krümmungslinie vor. Üblicherweise weist die Brillenscheibe eine horizontale Krümmungslinie auf, die sich bei bestimmungsgemäßem Gebrauch beispielsweise von einem Auge des Benutzers zum anderen Auge des Benutzers horizontal erstreckt, sodass die Brillenscheibe von einem Auge zum anderen Auge horizontal der Kopfform folgt und dadurch einen vorteilhaften Sitz für den Benutzer gewährleistet.

Damit den Augen während des Tragens der Schutzbrille ausreichend Luft aus der Umgebung zur Verfügung steht, um ein Beschlagen der Brillenscheibe zu vermeiden, und um ausreichend Raum für Wimpern des Benutzers zur Verfügung zu stellen, wird die eine Brillenscheibe üblicherweise auf Abstand zu Augen und Gesicht des Benutzers gehalten. Hierzu dient in der Regel eine Nasenauflage, die an der Brillenscheibe befestigt ist und auf der Nase des Benutzers derart positionierbar ist, dass ein Abstand der Brillenscheibe zu den Augen des Benutzers beziehungsweise zu dem Gesicht des Benutzers verbleibt. Somit besteht ein Zielkonflikt zwischen dem Vorhalten eines Abstands zur Vermeidung von Beschlag oder Wimpernanschlag einerseits und dem Schutz der Augen vor störenden Produkten aus der Umwelt andererseits.

Aus der Offenlegungsschrift US 5,825,455 ist bereits eine Schutzbrille mit zwei Brillenscheiben bekannt, die jeweils zwei Krümmungslinien aufweisen, entlang derer sich die jeweilige Brillenscheibe erstreckt. Auch die Offenlegungsschrift WO 2018/117943 A1 offenbart eine derartige Schutzbrille. Aus der Offenlegungsschrift WO 2008/072119 A1 ist eine Schutzbrille mit einer daran lösbar befestigbaren Dichtlippe bekannt.

Der Erfindung liegt die Aufgabe zugrunde, eine verbesserte Schutzbrille zu schaffen, die einen verbesserten Schutz der Augen trotz ausreichender Belüftung der Augen gewährleistet, ohne dass der Benutzungskomfort für den Benutzer beeinträchtigt wird.

Die der Erfindung zugrunde liegen der Aufgabe wird durch eine Schutzbrille mit den Merkmalen des Anspruchs 1 gelöst. Diese hat den Vorteil, dass der Brillenrand besonders nah zum Gesicht des Benutzers anordenbar ist und dennoch einen Abstand gewährleistet, der eine ausreichende Belüftung sowie einen hohen Tragekomfort erlaubt. Durch die erfindungsgemäße Ausgestaltung der Brillenscheibe der Schutzbrille wird dies ohne zusätzliche Hilfsmittel in kostengünstiger und für den Benutzer komfortsteigernder Art und Weise realisiert. Erfindungsgemäß ist dazu vorgesehen, jeder Brillenscheibenabschnitt eine erste und eine zweite Krümmungslinie aufweist, die zumindest im Wesentlichen quer zueinander ausgerichtet sind, und entlang welcher sich der jeweilige Brillenscheibenabschnitt erstreckt, und dass die Krümmungslinien des einen Brillenscheibenabschnitts geneigt zu den Krümmungslinien des anderen Brillenscheibenabschnitts ausgerichtet sind. Jeder Brillenscheibenabschnitt erstreckt sich somit entlang zweier quer zueinander ausgerichteter Krümmungslinien, insbesondere sodass sich in jedem Brillenscheibenabschnitt jeweils eine Wölbung ergibt, die bei bestimmungsgemäßem Gebrauch der Schutzbrille von dem Gesicht des Benutzers vorsteht. Jedem Auge ist dabei eine eigene Wölbung zugeordnet, wodurch der jeweilige Brillenscheibenabschnitt optimal an das Gesicht des Benutzers angepasst ist. Dadurch, dass die Krümmungslinien der beiden Brillenscheibenabschnitte geneigt zueinander ausgerichtet sind, ergibt sich, dass die Wölbungen verdreht oder verkippt zueinander ausgerichtet sind. Dadurch ist eine besonders vorteilhafte Anpassung der Brillenscheibenabschnitte an das Gesicht des Benutzers, unter Berücksichtigung der natürlichen Gesichtsform gewährleistet. Denn dadurch wird insbesondere erreicht, das entlang des gesamten Rands der Brillenscheibe der Abstand zum Gesicht des Benutzers zumindest im Wesentlichen konstant ist. Insbesondere sind die erste Krümmungslinie und die zweite Krümmungslinie in jeweils nur einer Dimension gekrümmt, sodass bei einer Draufsicht auf die Brillenscheibe die Krümmungslinien gradlinig verlaufen, in einer Zeichnung bei einer Draufsicht auf die Brillenscheibe also als gerade Linien dargestellt werden können, die sich schneiden. Durch die durch die zwei Krümmungslinien erreichte Wölbung der Brillenscheibenabschnitte ergibt sich außerdem der Vorteil, dass ein mittlerer Bereich des Scheibenbrillenabschnitts derart weit vom Auge des Benutzers beabstandet liegt, dass Wimpern des Benutzers nicht gegen Brillenscheibenabschnitt treffen. Durch die Wölbung wird die Brillenscheibe derart weit von den Augen des Benutzers wegbewegt, dass der Benutzer seine Augenlider bewegen, also schließen und öffnen kann, ohne dass Wimpern gegen das Material der Brillenscheibe stoßen. Lediglich am Randbereich liegt der jeweilige Brillenscheibenabschnitt nahe zum Gesicht des Benutzers. Dadurch ist der Komfort für den Benutzer deutlich erhöht. Dadurch, dass die Krümmungslinien des einen Brillenscheibenabschnitts geneigt zu den Krümmungslinien des anderen Brillenscheibenabschnitts ausgerichtet sind, ergibt sich, dass die erste Krümmungslinie nicht genau horizontal verläuft, sondern insbesondere geneigt zur Horizontalen ausgerichtet ist.

Gemäß einer bevorzugten Weiterbildung der Erfindung erstreckt sich die erste Krümmungslinie jeweils zwischen dem oberen und dem unteren Rand im Wesentlichen horizontal. Unter einer horizontalen Erstreckung ist dabei insbesondere eine solche Erstreckung zu verstehen, welche sich bei bestimmungsgemäßen Gebrauch von einem Augen des Benutzers in Richtung des anderen Auge des Benutzers erstreckt, also bei gerade ausgerichteten Kopf des Benutzers horizontal. Die erste Krümmungslinie gewährleistet somit, dass die Seitenränder der Brillenscheibe nah an dem Gesicht des Benutzers liegen und die Brillenscheibe der grundsätzlichen Krümmung des Kopfes des Benutzers in horizontaler Ebene folgt.

Besonders bevorzugt sind die ersten Krümmungslinien derart geneigt zueinander ausgerichtet, dass sie in Richtung des oberen Rands aufeinander zu laufen. Damit sind die Wölbungen der Brillenscheibenabschnitte vorteilhaft in Bezug auf den Nasenrücken des Benutzers ausgerichtet und laufen in Richtung des Nasenrückens schräg zueinander aufeinander zu. Dadurch erstrecken sich die Wölbungen besonders nahe bis zu einer Mittelhochachse der Brillenscheibe oberhalb des Nasenbereichs, sodass auch im Bereich der Nasenauflage eine vorteilhafte Anpassung der Brillenscheibe an das Gesicht des Benutzers gewährleistet ist.

Weiterhin erstreckt sich bevorzugt die zweite der Krümmungslinie jeweils von dem oberen Rand zu dem unteren Rand zumindest im Wesentlichen vertikal. Die zweite Krümmungslinie, die quer zu der ersten Krümmungslinie ausgerichtet ist, verläuft somit von dem oberen zu dem unteren Rand und damit bei bestimmungsgemäßen Gebrauch vertikal, wodurch sich eine Wölbung der Brillenscheibe ergibt, die von dem oberen Rand zu dem unteren Rand führt. Durch die Wölbung endet der untere Rand nah zu der Wange des Benutzers, wodurch der zuvor genannte Vorteil des verkürzten Abstandes erreicht wird. Die jeweilige zweite Krümmungslinie kann sich genau vertikal erstrecken, sodass die zweiten Krümmungslinien parallel zueinander ausgerichtet sind, vorzugsweise sind jedoch auch die zweiten Krümmungslinien geneigt zueinander ausgerichtet und stehen jeweils im gleichen Winkel zu ihrer zugehörigen ersten Krümmungslinie.

Bevorzugt liegt ein Schnittpunkt der ersten und zweiten Krümmungslinie eines Brillenscheibenabschnitts zumindest im Wesentlichen in der Mitte des jeweiligen Brillenscheibenabschnitts der Brillenscheibe. Dadurch ergibt sich eine vorteilhafte Ausrichtung der jeweiligen Wölbung des jeweiligen Brillenscheibenabschnitts in Bezug auf das Auge des Benutzers, was insbesondere zu optischen Vorteilen für den Benutzer beim Hindurchblicken durch die Brillenscheibe führt. Insbesondere hierdurch ergibt sich der zuvor genannte Vorteil des gleichmäßigen Abstands der Brillenscheibe zu dem Auge, insbesondere zu Wimpern des Benutzers.

Vorzugsweise werden die Brillenabschnitte der Schutzbrille durch separate Brillenscheiben ausgebildet, die miteinander, insbesondere durch eine Nasenauflage, fest verbunden sind. Dadurch sind eine kostengünstige Herstellung und eine einfache Montage der Schutzbrille mit einer optimalen Anpassung an das Gesicht des Benutzers möglich.

Alternativ weist die Schutzbrille eine durchgehende Brillenscheibe auf, welche beide Brillenscheibenabschnitte ausbildet. Dadurch ergibt sich eine besonders stabile Schutzbrille, die zwei Brillenscheibenabschnitte aufweist, die jeweils in zwei Richtungen gekrümmt und geneigt zueinander ausgebildet sind, wie vorstehend beschrieben.

Vorzugsweise schneidet die jeweilige zweite Krümmungslinie die jeweils zugeordnete erste Krümmungslinie senkrecht. Dadurch weist der jeweilige Brillenabschnitt einen Krümmungsmittelpunkt auf, in dem sich die beiden Krümmungslinien treffen und von dem auch die beiden Krümmungen senkrecht zueinander ausgerichtet sind. Es ergibt sich damit eine im Wesentlichen abschnittsweise kugelsegmentförmige Kontur der Brillenscheibe.

Erfindungsgemäß ist die jeweilige erste Krümmungslinie um 5° bis 30°, insbesondere um 10 bis 14°, bevorzugt um 12° geneigt zu einer Horizontalachse der Schutzbrille ausgerichtet. Hierdurch ergibt sich, dass sich die horizontale Krümmungslinie nicht genau horizontal erstreckt, sondern leicht geneigt zur Horizontalachse, sodass insbesondere am äußeren Seitenrand der Brille ein vorteilhafter der Brillenscheibe zu dem Gesicht des Benutzers im Bereich der Wange gewährleistet ist. Insbesondere sind die ersten Krümmungslinien an einer vertikal Mittelachse der Schutzbrille gespiegelt zueinander ausgebildet, sodass die Schutzbrille insgesamt spiegelsymmetrisch ist.

Weiterhin ist erfindungsgemäß vorgesehen, dass die erste Krümmungslinie jeweils einen Radius von 43 mm bis 63 mm, insbesondere 50 mm bis 56 mm, vorzugsweise 53 mm aufweist. Dadurch ergibt sich eine vorteilhafte Krümmung, die für den größten Anteil der menschlichen Bevölkerung einen vorteilhaften Sitz der Schutzbrille auf dem Gesicht gewährleistet.

Erfindungsgemäß weist die jeweilige zweite Krümmungslinie einen Radius von 30 mm bis 50 mm, insbesondere 35 mm bis 45 mm, vorzugsweise 40 mm auf. Dies gewährleistet ein vorteilhaft nahes Heranführen des insbesondere des unteren Rands der Brillenscheibe an das Gesicht des Benutzers, um einen ausreichenden Luftspalt bei bestmöglichem Schutz und Tragekomfort zu gewährleisten.

Gemäß einer bevorzugten Weiterbildung der Erfindung weist die Brillenscheibe an ihrem unteren Rand eine Nasenauflage auf, wie vorstehend bereits schon erwähnt. Bevorzugt erstreckt sich eine elastisch verformbare Schutzlippe nur an dem unteren Rand der Brillenscheibe von der Nasenauflage aus in Richtung zumindest eines der äußeren Seitenränder der Brillenscheibe und von dem unteren Rand der Brillenscheibe in Verlängerung der Brillenscheibe nach unten zur Auflage auf der Wange des Benutzers. Durch die Schutzlippe wird erreicht, dass im Wangenbereich der Spalt oder die Lücke zwischen der Brillenscheibe und der Wange des Benutzers überbrückt wird und dadurch das Eindringen von beispielsweise Schmutzpartikeln in den Raum zwischen Brillenscheibe und Auge des Benutzers vollständig verhindert wird. Dadurch, dass sich die Schutzlippe nur an dem unteren Rand der Brillenscheibe erstreckt, ist gewährleistet, dass im Bereich der Seitenränder sowie des oberen Rands ausreichen Austausch mit der Umgebungsluft stattfindet, die beispielsweise ein Beschlagen der Brillenscheibe verhindert. Dadurch, dass sich die Schutzlippe in Verlängerung der Brillenscheibe nach unten zur Auflage auf der Wange des Benutzers erstreckt, entsteht eine Schutzlippe, welche auf der Wange des Benutzers insbesondere flächig aufliegt und sich in Richtung eines Bereichs erstreckt, in welchem ansonsten eine Atemmaske angeordnet werden kann. Die Atemmaske ist dabei optional auf der Schutzlippe platzierbar, sodass sich Schutzlippe und Atemmaske überlappen und zum einen das Gesicht und die Augen des Benutzers schützen und zum anderen eine sichere Auflage der Schutzlippe auf dem Gesicht des Benutzers einerseits sowie der Atemmaske auf dem Gesicht des Benutzers andererseits gewährleisten. Durch die vorteilhafte Ausbildung der Schutzbrille wird erreicht, dass die Brillenscheibe besonders nah zum Gesicht des Benutzers anordenbar ist und dadurch auch die Atemmaske besonders nah zu dem Augenbereich des Benutzers anordenbar ist, wodurch sich ein vorteilhafter Sitz der Atemschutzmaske auf dem Gesicht des Benutzers ergibt. Dadurch wird beispielsweise auch eine Kollisionsgefahr zwischen der Schutzbrille und einer verwendeten Atemschutzmaske reduziert, sodass beide bequem ohne Komforteinbußen getragen werden können.

Gemäß einer bevorzugten Weiterbildung der Erfindung bildet die Schutzlippe die Nasenauflage der Schutzbrille mit oder aus. Damit ist entweder für jedes Auge des Benutzers eine eigene Schutzlippe vorhanden, die insbesondere an einer separaten Brillenscheibe angeformt oder befestigt ist, oder die Schutzlippe erstreckt sich über den gesamten unteren Rand der Schutzbrille über beide Brillenscheibenabschnitte hinweg und bildet dadurch mittig die Nasenauflage mit aus. Die Schutzlippe ist somit einstückig mit der Nasenauflage ausgebildet. Vorzugsweise ist die Nasenauflage zumindest im Wesentlichen auf der vom Gesicht des Benutzers abgewandten Seite der Schutzbrille ausgebildet, wodurch die Brillenscheiben besonders nah zu dem Gesicht des Benutzers anordenbar sind. Gemäß einer Ausführungsform erstreckt sich dazu die Schutzlippe ausgehend von dem unteren Rand der Brillenscheibe gerade in einer Ebene nach unten, wobei sie die Krümmung gemäß der ersten horizontalen Krümmungslinie mitführt. Alternativ weist die Schutzlippe eine eigene Krümmung um eine Horizontalachse auf, die dazu führt, dass eine Vorbiegung der Schutzlippe derart erfolgt, dass die Schutzlippe flächig auf der Wange zum Aufliegen kommt.

Das erfindungsgemäße Schutzsystem mit den Merkmalen des Anspruchs 14 zeichnet sich durch die erfindungsgemäße Ausbildung der Schutzbrille aus. Es ergeben sich dadurch zumindest die zuvor bereits genannten Vorteile. Darüber hinaus wird durch die vorteilhafte Ausbildung der Schutzbrille erreicht, dass die Schutzmaske besonders nahe zu den Augen des Benutzers beziehungsweise zu der Schutzbrille angeordnet werden kann und dadurch einen hohen Tragekomfort gewährleistet. Insbesondere ist die Schutzbrille an die Form der Atemschutzmaske angepasst, um einen optimalen Sitz beider auf dem Gesicht des Benutzers zu gewährleisten, ohne dass diese während des Tragens aneinander stoßen oder miteinander derart kollidieren, dass ein optimaler Sitz des jeweiligen Teils verhindert ist.

Besonders bevorzugt weist die Schutzbrille die zuvor erwähnte Schutzlippe auf, wobei die Schutzlippe vorzugsweise einen Maskenauflagenbereich aufweist. Insbesondere ist der Atemmaskenauflagebereich als Gleitbereich ausgebildet, sodass ein Bewegen der Atemmaske auf dem Gesicht des Benutzers sich nicht zu einem Bewegen der Schutzbrille führt.

Im Folgenden soll die Erfindung anhand der Zeichnung näher erläutert werden. Dazu zeigen
- Figur 1: eine vorteilhafte Schutzbrille in einer perspektivischen Darstellung,
- Figur 2: eine perspektivische Draufsicht auf die Schutzbrille von oben,
- Figur 3: eine Querschnittsdarstellung der Schutzbrille und
- Figur 4: eine Draufsicht auf die Schutzbrille von vorne.

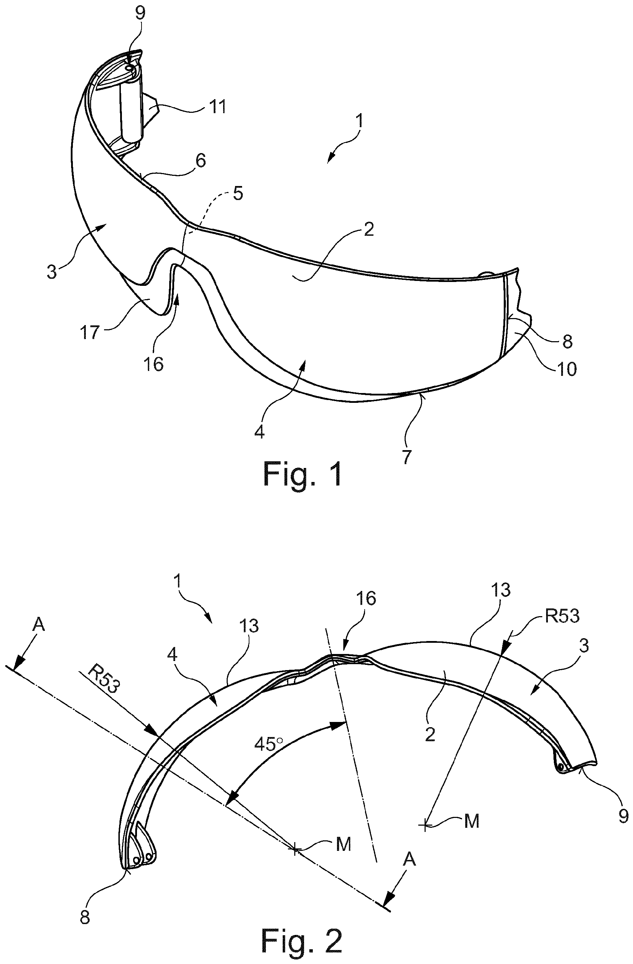

Figur 1 zeigt in einer perspektivischen Darstellung eine vorteilhafte Schutzbrille 1 für einen menschlichen Benutzer. Die Schutzbrille 1 weist eine insbesondere aus einem durchsichtigen Material gefertigte Brillenscheibe 2 auf, die zwei Brillenscheibenabschnitte 3, 4 bildet, wobei jeweils ein Brillenscheibenabschnitt 3, 4 einem Auge des Benutzers zuordenbar ist. Die Brillenscheibe 2 ist als durchgehende Brillenscheibe ausgebildet, die beide Brillenscheibenabschnitte 3, 4 mitbildet. Eine alternative Ausgestaltung, bei welcher der jeweilige Brillenscheibenabschnitt 3, 4 durch eine einzelne Brillenscheibe 2 gebildet ist, ist gemäß einem weiteren Ausführungsbeispiel vorgesehen und in Figur 1 durch eine gestrichelte Mitteltrennlinie 5 angedeutet.

Die Brillenscheibe 2 weist einen der Stirn des Benutzers zuordenbaren oberen Rand 6, einen der Wangen des Benutzers zuordenbaren unteren Rand 7 sowie zwei äußere Seitenränder 8 und 9 auf, an welche sich jeweils ein Brillenbügel 10, 11 zur Auflage auf einem Ohr des Benutzers anschließt. Die Brillenbügel 10, 11 sind hier nur abschnittsweise gezeigt.

Die Brillenscheibe 2 der vorteilhaften Schutzbrille 1 ist derart ausgebildet, dass sich jeder Brillenscheibenabschnitt 3, 4 entlang von zwei Krümmungslinien erstreckt, die jeweils quer, insbesondere senkrecht zueinander ausgerichtet sind.

Figur 2 zeigt hierzu eine Draufsicht von oben auf die Schutzbrille 1. In der Draufsicht ist erkennbar, dass beide Brillenscheibenabschnitte 3, 4 eine erste Krümmung aufweisen. Diese wird dadurch erreicht, dass der jeweilige Brillenscheibenabschnitt 3, 4 sich entlang einer ersten Krümmungslinie 13 erstreckt, die sich im Wesentlichen horizontal entlang der Brillenscheibe 2 erstreckt. Die Krümmungslinien 13 sind dabei konvex bezogen auf einen Krümmungsmittelpunkt M ausgebildet, der bei bestimmungsgemäßem Gebrauch im Auge des Benutzers liegt. Vorliegend weist die jeweilige erste Krümmungslinie 13 einen Radius von 53 mm auf, wobei in Figur 2 nur der Radius für den Brillenabschnitt 4 eingezeichnet ist.

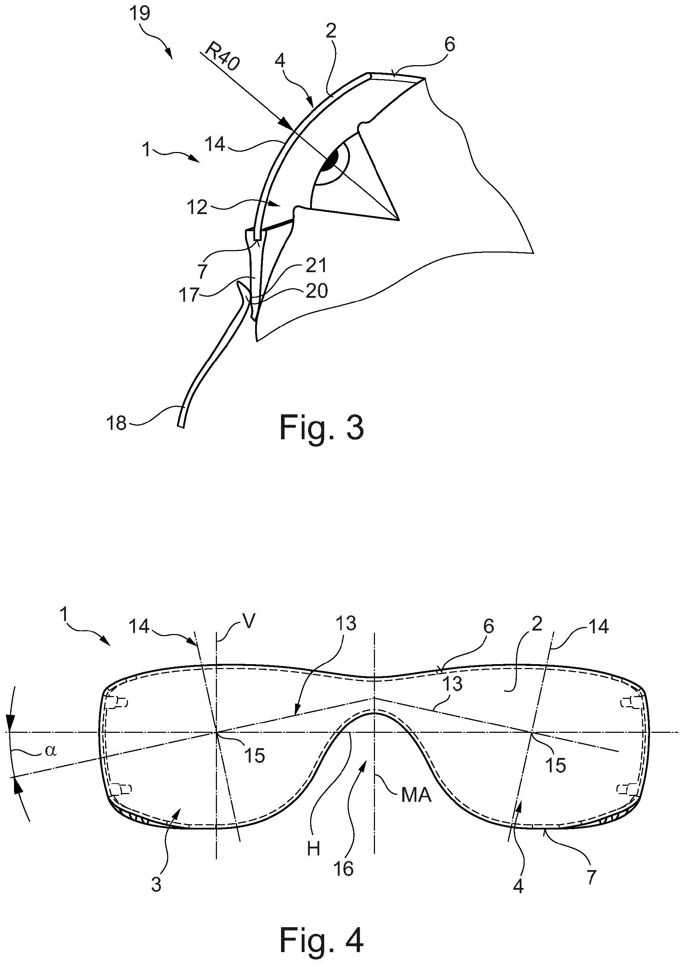

Figur 3 zeigt eine Querschnittsdarstellung durch die Schutzbrille 1 aus Figur 2 entlang der Linie A-A. Durch die Querschnittsdarstellung ist gut erkennbar, dass die Brillenscheibe 2, insbesondere der Brillenscheibenabschnitt 4, eine zweite Krümmungslinie 14 aufweist, die sich quer zu der Krümmungslinie 13 erstreckt. Außerdem zeigt Figur 3 beispielhaft die Lage der Schutzbrille 1 zu einem Auge 12 eines hier nicht näher dargestellten Benutzers.

Figur 4 zeigt hierzu eine Draufsicht auf die Vorderseite der Schutzbrille 1. Durch gestrichelte Linien sind die Krümmungslinien 13, 14 eingezeichnet für den Brillenscheibenabschnitt 4, die sich in einem Punkt 15 etwa mittig in dem Brillenabschnitt 4 kreuzen und vorliegend senkrecht zueinander ausgerichtet sind. Die Krümmungslinien 13, 14 sind jeweils in nur einer Dimension gekrümmt, sodass sie in der in der Figur gezeigten Draufsicht gradlinig erscheinen. Figur 4 zeigt insoweit Projektionen der Krümmungslinien 13, 14. Außerdem ist die erste Krümmungslinie 13 um einen Winkel α von vorliegend α = 12° zu einer Horizontalen H geneigt, sodass die Krümmungslinie 13 in der Draufsicht gegen den Uhrzeigersinn um den Winkel α verdreht ausgerichtet ist. Das führt dazu, dass der Schnittpunkt 15 etwa mittig in dem Brillenscheibenabschnitt 4 liegt und die Krümmungslinie 13 im Übergang zu dem benachbarten Brillenscheibenabschnitt 3 oberhalb einer eine Nasenauflage 16 bildende Einbuchtung am unteren Rand 7 der Brillenscheibe 2 verläuft. Weil die Krümmungslinie 14 senkrecht zur Krümmungslinie 13 ausgerichtet ist, ist auch die zweite Krümmungslinie 14 geneigt, insbesondere zu einer Vertikalachse V ausgerichtet.

Die erste und zweite Krümmungslinie 13, 14, die dem Brillenscheibenabschnitt 3 zugeordnet sind, sind spiegelbildlich zu den Krümmungslinien 13, 14 des Brillenscheibenabschnitts 4 ausgerichtet, wobei hierzu eine Spiegelung an einer vertikalen Mittelachse MA stattfindet, die in Figur 4 vertikal mittig durch die Brillenscheibe 2, entsprechend der beispielhaften Trennlinie 5 verläuft.

Die Brillenscheibenabschnitte 3, 4, die sich nur entlang der Krümmungslinien 13 und 14 erstrecken, sind somit in zwei unterschiedliche Richtungen gewölbt ausgebildet, sodass sie zumindest abschnittsweise kugelsegmentförmig gestaltet sind. Vorliegend weist dabei die jeweils erste Krümmungslinie 13 einen Radius von 53 mm (R53) auf. Die jeweilige zweite Krümmungslinie 14 weist hingegen jeweils einen bevorzugten Radius von 40 mm (R40) auf.

Durch den vorteilhaften Verlauf der Krümmungslinie 13 wird erreicht, dass die Brillenscheibe 2 das Gesicht des Benutzers vorteilhaft seitlich umfasst. Durch den vorteilhaften Verlauf der Krümmungslinie 14 wird erreicht, dass der jeweilige Brillenscheibenabschnitt 3, 4 sich vorteilhaft von der Stirn des Benutzers, also vom oberen Rand 6, bis zu der Wange des Benutzers, also bis zum unteren Rand 7, derart erstreckt, dass die Brillenscheibe sowohl am oberen Rand als auch am unteren Rand 7 einen nur kleinen Abstand zu dem Gesicht des Benutzers aufweist. Durch den kleinen Abstand wird gewährleistet, dass einerseits ein ausreichender Luftaustausch stattfinden kann, der ein Beschlagen der Brillenscheibe 2 verhindert, und dass andererseits der Abstand klein genug ist, um die Augen des Benutzers vor beispielsweise Spänen oder Flüssigkeit, die bei der Bearbeitung eines Werkstücks in Richtung des Benutzers geschleudert werden können, geschützt sind. Insbesondere durch die zweite Krümmung entlang der Krümmungslinie 14 wird erreicht, dass sich der jeweilige Brillenscheibenabschnitt 3, 4 vor dem jeweiligen Auge 12 des Benutzers derart wölbt, dass am Randbereich der erwähnte kleine Abstand gewährleistet ist, und im mittleren Bereich ein ausreichend großer Abstand zum Auge 12 besteht, der verhindert, dass der Benutzer beim Tragen der Schutzbrille 1 beispielsweise mit seinen Wimpern gegen die Brillenscheibe 2 stößt, wie in Figur 3 angedeutet. Durch die vorteilhafte doppelgewölbte Ausbildung der Brillenscheibenabschnitte 3, 4 wird somit Raum für die Bewegung der Augenlider und Wimpern geschaffen, ohne dass dadurch ein großer Abstand zwischen der Brillenscheibe 2 in ihrem Rand und dem Gesicht des Benutzers entsteht.

Durch die Neigung der ersten Krümmungslinien 13 zueinander derart, dass sie in Richtung des oberen Rands 6 aufeinander zu laufen, also insbesondere einen stumpfen Winkel zwischen sich einschließen, wird erreicht, dass sich die jeweilige Wölbung des jeweiligen Brillenscheibenabschnitts 3, 4 vorteilhaft in die Nasenregion beziehungsweise in Richtung der Nasenauflage der Bremsscheibe 2 erstreckt. Insbesondere enden damit die ersten Krümmungslinien 13 oberhalb der Nase beziehungsweise der Nasenauflage, sodass auch in diesem Bereich eine vorteilhafte Anpassung des Brillenscheibenabschnitts an die Gesichtsform des Benutzers gewährleistet ist.

Um den ohnehin kleinen Abstand, der zwischen dem unteren Rand 7 und der Wange des Benutzers verbleibt, noch weiter zu reduzieren, weist die Schutzbrille 1 optional eine Schutzlippe 17 auf. Die Schutzlippe 17 erstreckt sich nur entlang des unteren Rands 7, vorliegend einstückig von dem Brillenscheibenabschnitt 4 zu dem Brillenscheibenabschnitt 3, sodass sie die Nasenauflage 16 mitbildet. Außerdem erstreckt sich die Schutzlippe 17 in der Verlängerung der Brillenscheibe 2 nach unten, also von dem unteren Rand 7 aus weiter in Verlängerung der Brillenscheibe, sodass die Schutzlippe 17 bei bestimmungsgemäßem Gebrauch auf der Wange des Benutzers zum Liegen kommt und dadurch die Lücke zwischen dem unteren Rand 7 und der Wange des Benutzers vollständig schließt beziehungsweise abdichtet, sodass in diesem Bereich ein Hindurchtreten von Schmutzpartikeln, Späne, Flüssigkeit oder dergleichen sicher verhindert ist. Optional ist die Schutzlippe 17 aus einem durchsichtigen Material gefertigt, um die Sicht des Benutzers nicht weiter einzuschränken. Insbesondere ist die Schutzlippe 17 direkt an die Brillenscheibe 2 angeformt und insbesondere stoffschlüssig mit dieser verbunden, um eine dauerhaft feste Anordnung der Schutzlippe 17 an der Brillenscheibe 2 zu gewährleisten.

Vorteilhafterweise ist die Schutzlippe 17 derart ausgebildet, dass sie die Nasenauflage 16 derart bildet, dass die Nasenauflage 16 zumindest im Wesentlichen auf der von dem Gesicht des Benutzers abgewandten Seite der Brillenscheibe 2, also von der Brillenscheibe 2 nach außen vorstehend, ausgebildet ist. Dadurch ist die Brillenscheibe 2 besonders nahe zum Gesicht des Benutzers anordenbar.

Während gemäß dem vorliegenden Ausführungsbeispiel die ersten Krümmungslinien 13 um einen Winkel α von 12° zu der Horizontalen H geneigt sind, ist grundzusätzlich festzustellen, dass sich eine Neigung von 5° bis 30°, insbesondere von 10° bis 14° grundsätzlich für die vorteilhafte Ausbildung der Schutzbrille eignet. Entsprechendes gilt auch für die genannten Radien, die hier mit 53 mm für die erste Krümmungslinie und 40 mm für die zweite Krümmungslinie in dem Ausführungsbeispiel angegeben sind. Grundsätzlich können die Radien jedoch von diesen genannten Radien auch bis zu einem gewissen Grad abweichen. Insbesondere zeigen sich Radien für die erste Krümmungslinie 13 von 43 bis 63 mm, insbesondere von 50 bis 56 mm als vorteilhaft. Für die jeweilige zweite Krümmungslinie 14 sind Radien von 30 bis 50 mm, insbesondere von 35 bis 45 mm von Vorteil.

Optional wird die Schutzbrille 1 durch eine Atemschutzmaske 18 zu einem Schutzsystem 19 ergänzt, wie es in Figur 3 angedeutet ist. Die Atemschutzmaske 18 ist dabei dazu ausgebildet, sich bei bestimmungsgemäßem Gebrauch über Mund und Nase des Benutzers zu erstrecken und an ihrem Außenrand 20 dichtend auf dem Gesicht des Benutzers aufzuliegen. Ist die Schutzlippe 17 vorhanden, so dient diese zumindest als bereichsweise Auflage für den Außenrand 20 der Atemmaske 18. Insbesondere weist die Schutzlippe 17 dazu einen vom Gesicht des Benutzers abgewandten Gleitabschnitt 21 auf, auf welchem die Atemmaske 18 mit ihrem Außenrand 20 auflegbar ist. Der Gleitabschnitt 21 bewirkt, dass sich die Atemmaske 18 auf der Schutzlippe 17 verschieben lässt, ohne dass dadurch auch die Schutzbrille 1 auf dem Gesicht des Benutzers verschoben wird.

Durch die vorteilhafte Ausbildung von Atemschutzmaske 18 und Schutzbrille 1 wird erreicht, dass dadurch insgesamt ein hoher Tragekomfort für das gesamt Schutzsystem 19 erzielt wird.

## Patentansprüche

1. Schutzbrille (1) für einen menschlichen Benutzer, mit zumindest einer Brillenscheibe (2), die einen der Stirn des Benutzers zuordenbaren oberen Rand (6) und einen der Wange des Benutzers zuordenbaren unteren Rand (7) und für jedes Auge des Benutzers jeweils einen Brillenscheibenabschnitt (3,4) aufweist, der sich jeweils entlang zumindest einer Krümmungslinie (13,14) erstreckt, wobei jeder Brillenscheibenabschnitt (3,4) eine erste und eine zweite Krümmungslinie (14) aufweist, die zumindest im Wesentlichen quer zueinander ausgerichtet sind, und entlang welcher sich der jeweilige Brillenscheibenabschnitt (3,4) erstreckt, und wobei die Krümmungslinien (13,14) des einen Brillenscheibenabschnitts (3) geneigt zu den Krümmungslinien (13,14) des anderen Brillenabschnitts (4) ausgerichtet sind, **dadurch gekennzeichnet, dass** die erste Krümmungslinie (13) jeweils einen Radius von 43 mm bis 63 mm und die zweite Krümmungslinie (14) jeweils einen Radius von 30 mm bis 50 mm aufweist, und die jeweilige erste Krümmungslinie (13) um 5° bis 30°geneigt zu einer Horizontalachse (H) der Schutzbrille (1) ausgerichtet ist.

2. Schutzbrille nach Anspruch 1, **dadurch gekennzeichnet, dass** sich die jeweils erste Krümmungslinie (13) zwischen dem oberen Rand (6) und dem unteren Rand (7) der Brillenscheibe (2) im Wesentlichen horizontal erstreckt.

3. Schutzbrille nach einem der vorhergehenden Ansprüche, **dadurch gekennzeichnet, dass** die ersten Krümmungslinien (13) derart geneigt zueinander ausgerichtet sind, dass sie in Richtung des oberen Rands (6) aufeinander zu laufen.

4. Schutzbrille nach einem der vorhergehenden Ansprüche, **dadurch gekennzeichnet, dass** sich die zweite Krümmungslinie (14) von dem oberen Rand (6) zu dem unteren Rand (7) im Wesentlichen vertikal erstreckt.

5. Schutzbrille nach einem der vorhergehenden Ansprüche, **dadurch gekennzeichnet, dass** ein Schnittpunkt (15) der ersten und zweiten Krümmungslinie (13,14) jedes Brillenscheibenabschnitts (3,4) zumindest im Wesentlichen in der Mitte des jeweiligen Brillenscheibenabschnitts (3,4) der Brillenscheibe (2) liegt.

6. Schutzbrille nach einem der vorhergehenden Ansprüche, **dadurch gekennzeichnet, dass** der jeweilige, einem Auge (12) zugeordnete Brillenscheibenabschnitt (3,4) der Brillenscheibe (2) als separate Brillenscheibe (2) ausgebildet ist.

7. Schutzbrille nach einem der Ansprüche 1 bis 5, **dadurch gekennzeichnet, dass** die Brillenscheibe (2) durchgehend beide Brillenscheibenabschnitte (3,4) einstückig ausbildet.

8. Schutzbrille nach einem der vorhergehenden Ansprüche, **dadurch gekennzeichnet, dass** die jeweilige zweite Krümmungslinie (14) die jeweils zugeordnete erste Krümmungslinie (13) senkrecht schneidet.

9. Schutzbrille nach einem der vorhergehenden Ansprüche, **dadurch gekennzeichnet, dass** die jeweilige erste Krümmungslinie (13) um 10° bis 14°, bevorzugt um 12°, geneigt zu einer Horizontalachse (H) der Schutzbrille (1) ausgerichtet ist.

10. Schutzbrille nach einem der vorhergehenden Ansprüche, **dadurch gekennzeichnet, dass** die erste Krümmungslinie (13) jeweils einen Radius von 50 mm bis 56 mm, bevorzugt 53 mm, aufweist.

11. Schutzbrille nach einem der vorhergehenden Ansprüche, **dadurch gekennzeichnet, dass** die zweite Krümmungslinie (14) jeweils einen Radius von 35 mm bis 45 mm, bevorzugt 40 mm, aufweist.

12. Schutzbrille nach einem der vorhergehenden Ansprüche, **dadurch gekennzeichnet, dass** die Brillenscheibe (2) an dem unteren Rand (7) eine Nasenauflage (16) aufweist, und dass sich eine elastisch verformbare Schutzlippe (17) nur an dem unteren Rand (7) der Brillenscheibe (2) von der Nasenauflage (16) aus in Richtung zumindest eines äußeren Seitenrands (8,9) der Brillenscheibe (2) und von dem unteren Rand (7) der Brillenscheibe (2) in Verlängerung der Brillenscheibe (2) nach unten zur Auflage auf der Wange des Benutzers erstreckt.

13. Schutzbrille nach Anspruch 12, **dadurch gekennzeichnet, dass** die Schutzlippe (17) die Nasenauflage (16) bildet oder mitbildet.

14. Schutzsystem (19) für einen menschlichen Benutzer, mit einer Atemschutzmaske (18) und mit einer Schutzbrille (1), **gekennzeichnet durch** die Ausbildung der Schutzbrille (1) gemäß einem der Ansprüche 1 bis 13.

15. Schutzsystem nach Anspruch 14 in Kombination mit Anspruch 12, **dadurch gekennzeichnet, dass** die Schutzlippe (17) einen Gleitbereich (21) aufweist, auf welchem die Atemschutzmaske (18) bereichsweise auflegbar oder aufgelegt ist.

## Claims

1. Protective eyewear (1) for a human user, with at least one lens (2) comprising an upper edge (6) that is assignable to the user's forehead, a lower edge (7) that is assignable to the user's cheek and a respective lens section (3,4) for each eye of the user, wherein the lens sections (3,4) each extend along at least one line of curvature (13,14), wherein each lens section (3,4) has a first line of curvature (13) and a second line of curvature (14) that are oriented at least substantially transversely to one another, and along which the respective lens section (3,4) extends, and that the lines of curvature (13,14) of one lens section (3) are inclined with respect to the lines of curvature (13, 14) of the other lens section (4), **characterised in that** each first line of curvature (13) has a radius of 43 mm to 63 mm and each second line of curvature (14) has a radius of 30 mm to 50 mm, and each first line of curvature (13) is inclined with respect to a horizontal axis (H) of the protective eyewear (1) at an angle of 5° to 30°.

2. Protective eyewear according to claim 1, **characterised in that** the respective first line of curvature (13) extends substantially horizontally between the upper edge (6) and the lower edge (7) of the lens (2).

3. Protective eyewear according to any of the preceding claims, **characterised in that** the first lines of curvature (13) are inclined relative to one another in such a way that they converge in the direction of the upper edge (6).

4. Protective eyewear according to any of the preceding claims, **characterised in that** the second line of curvature (14) extends substantially vertically from the upper edge (6) to the lower edge (7).

5. Protective eyewear according to any of the preceding claims, **characterised in that** an intersection point (15) of the first and second line of curvature (13,14) of each lens section (3,4) lies at least substantially in the middle of the respective lens section (3,4) of the lens (2).

6. Protective eyewear according to any of the preceding claims, **characterised in that** the respective lens section (3,4) of the lens (2) that is assigned to one eye (12), is embodied as a separate lens (2).

7. Protective eyewear according to any of claims 1 to 5, **characterised in that** both lens sections (3,4) are formed integrally by one continuous lens (2).

8. Protective eyewear according to any of the preceding claims, **characterised in that** each second line of curvature (14) intersects the corresponding first line of curvature (13) in a vertical fashion.

9. Protective eyewear according to any of the preceding claims, **characterised in that** each first line of curvature (13) is inclined with respect to a horizontal axis (H) of the protective eyewear (1) at an angle of 10° to 14° and preferably at an angle of 12°.

10. Protective eyewear according to any of the preceding claims, **characterised in that** each first line of curvature (13) has a radius of 50 mm to 56 mm and preferably of 53 mm.

11. Protective eyewear according to any of the preceding claims, **characterised in that** each second line of curvature (14) has a radius of 35 mm to 45 mm and preferably of 40 mm.

12. Protective eyewear according to any of the preceding claims, **characterised in that** the lens (2) has a nose support (16) on the lower edge (7) and that an elastically deformable protective lip (17) extends only on the lower edge (7) of the lens (2) from the nose support (16) in the direction of at least one outer side edge (8, 9) of the lens (2) and from the lower edge (7) of the lens (2) as an extension of the lens to be downwards supported on the user's cheek.

13. Protective eyewear according to claim 12, **characterised in that** the protective lip (17) forms or contributes to forming the nose support (16).

14. Protective system (19) for a human user, with a respiratory mask (18) and protective eyewear (1), **characterised in that** the protective eyewear (1) is configured in accordance with any of claims 1 to 13.

15. Protective system according to claim 14 in combination with claim 12, **characterised in that** the protective lip (17) has a sliding zone (21), wherein the respiratory mask (18) can be or is supported in regions on said sliding zone.

## Revendications

1. Lunettes de protection (1) pour un utilisateur humain, avec au moins un verre de lunettes (2), lequel présente un bord supérieur (6) pouvant être associé au front de l'utilisateur et un bord inférieur (7) pouvant être associé à la joue de l'utilisateur et pour chaque oeil de l'utilisateur respectivement une section de verre de lunettes (3, 4) qui s'étend respectivement le long d'au moins une ligne de courbure (13, 14), dans lesquelles chaque section de verre de lunettes (3, 4) présente une première et une deuxième ligne de courbure (14) qui sont orientées de manière au moins essentiellement transversale l'une par rapport à l'autre, et le long desquelles s'étend la section de verre de lunettes (3, 4) respective, et dans lesquelles les lignes de courbure (13, 14) de l'une section de verre de lunettes (3) sont orientées de manière inclinée par rapport aux lignes de courbure (13, 14) de l'autre section de lunettes (4), **caractérisées en ce que** la première ligne de courbure (13) présente respectivement un rayon allant de 43 mm à 63 mm et la deuxième ligne de courbure (14) présente respectivement un rayon allant de 30 mm à 50 mm, et la première ligne de courbure (13) respective est orientée de manière inclinée de 5° à 30° par rapport à un axe horizontal (H) des lunettes de protection (1).

2. Lunettes de protection selon la revendication 1, **caractérisées en ce que** la première ligne de courbure (13) respective s'étend de manière essentiellement horizontale entre le bord supérieur (6) et le bord inférieur (7) du verre de lunettes (2).

3. Lunettes de protection selon l'une des revendications précédentes, **caractérisées en ce que** les premières lignes de courbure (13) sont orientées de manière inclinée l'une par rapport à l'autre de telle sorte qu'elles convergent l'une vers l'autre en direction du bord supérieur (6).

4. Lunettes de protection selon l'une des revendications précédentes, **caractérisées en ce que** la deuxième ligne de courbure (14) s'étend de manière essentiellement verticale depuis le bord supérieur (6) jusqu'au bord inférieur (7).

5. Lunettes de protection selon l'une des revendications précédentes, **caractérisées en ce qu'**un point d'intersection (15) de la première et de la deuxième ligne de courbure (13, 14) de chaque section de verre de lunettes (3, 4) se situe au moins essentiellement au milieu de la section de verre de lunettes (3, 4) respective du verre de lunettes (2).

6. Lunettes de protection selon l'une des revendications précédentes, **caractérisées en ce que** la section de verre de lunettes (3, 4) respective du verre de lunettes (2) associée à un oeil (12) est conçue en tant que verre de lunettes (2) distinct.

7. Lunettes de protection selon l'une des revendications 1 à 5, **caractérisées en ce que** le verre de lunettes (2) forme de manière continue les deux sections de verre de lunettes (3, 4) en une seule pièce.

8. Lunettes de protection selon l'une des revendications précédentes, **caractérisées en ce que** la deuxième ligne de courbure (14) respective coupe perpendiculairement la première ligne de courbure (13) associée respectivement.

9. Lunettes de protection selon l'une des revendications précédentes, **caractérisées en ce que** la première ligne de courbure (13) respective est orientée de manière inclinée de 10° à 14°, de préférence de 12°, par rapport à un axe horizontal (H) des lunettes de protection (1).

10. Lunettes de protection selon l'une des revendications précédentes, **caractérisées en ce que** la première ligne de courbure (13) présente respectivement un rayon allant de 50 mm à 56 mm, de préférence de 53 mm.

11. Lunettes de protection selon l'une des revendications précédentes, **caractérisées en ce que** la deuxième ligne de courbure (14) présente respectivement un rayon allant de 35 mm à 45 mm, de préférence de 40 mm.

12. Lunettes de protection selon l'une des revendications précédentes, **caractérisées en ce que** le verre de lunettes (2) présente au niveau du bord inférieur (7) un patin de nez (16), et **en ce qu'**une lèvre de protection (17) élastiquement déformable uniquement au niveau du bord inférieur (7) du verre de lunettes (2) s'étend depuis le patin de nez (16) en direction d'au moins un bord latéral externe (8, 9) du verre de lunettes (2) et depuis le bord inférieur (7) du verre de lunettes (2) dans le prolongement du verre de lunettes (2) vers le bas pour permettre l'appui sur la joue de l'utilisateur.

13. Lunettes de protection selon la revendication 12, **caractérisées en ce que** la lèvre de protection (17) forme le patin de nez (16) ou forme un ensemble avec celui-ci.

14. Système de protection (19) pour un utilisateur humain, avec un masque de protection respiratoire (18) et avec des lunettes de protection (1), **caractérisé par** la conception des lunettes de protection (1) selon l'une des revendications 1 à 13.

15. Système de protection selon la revendication 14 en combinaison avec la revendication 12, **caractérisé en ce que** la lèvre de protection (17) présente une zone de glissement (21) sur laquelle peut être en appui ou est en appui par endroits le masque de protection respiratoire (18).
